(19) Europäisches Patentamt
European Patent Office
Office européen des brevets

(11) Veröffentlichungsnummer : **0 032 251**
**B1**

(12) # EUROPÄISCHE PATENTSCHRIFT

(45) Veröffentlichungstag der Patentschrift : **14.09.83**

(51) Int. Cl.³ : **C 08 G 18/62**, A 61 K 31/79

(21) Anmeldenummer : **80108257.9**

(22) Anmeldetag : **31.12.80**

(54) **Vinylpyrrolidonpolymerisate, ihre Herstellung, ihre Verwendung zur Herstellung von Blutersatzflüssigkeiten und diese Blutersatzflüssigkeiten.**

(30) Priorität : **12.01.80 DE 3001013**

(43) Veröffentlichungstag der Anmeldung : **22.07.81 Patentblatt 81/29**

(45) Bekanntmachung des Hinweises auf die Patenterteilung : **14.09.83 Patentblatt 83/37**

(84) Benannte Vertragsstaaten : **BE CH DE FR GB LI NL SE**

(56) Entgegenhaltungen :
**DE A 1 418 995**
**DE A 2 309 894**
**DE A 2 759 150**
**DE A 2 831 335**

(73) Patentinhaber : **BASF Aktiengesellschaft**
**Carl-Bosch-Strasse 38**
**D-6700 Ludwigshafen (DE)**

(72) Erfinder : **Straub, Ferdinand, Dr.-Chem.**
**Ziegelstrasse 20**
**D-6832 Hockenheim (DE)**
Erfinder : **Lang, Siegfried, Dr.**
**Thomas-Mann-Strasse 22**
**D-6700 Ludwigshafen (DE)**
Erfinder : **Roske, Eckhard**
**Hillensheimer Strasse 4**
**D-6700 Ludwigshafen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 032 251 B1

# Vinylpyrrolidonpolymerisate, ihre Herstellung, ihre Verwendung zur Herstellung von Blutersatzflüssigkeiten und diese Blutersatzflüssigkeiten

Für die erste Hilfe beim physiologischen Schock ist man häufig auf Blutersatzflüssigkeiten (Blutplasmaersatz) angewiesen, da Blutkonserven meist nicht sofort zur Verfügung stehen. Außerdem muß man ohne Verträglichkeitsrisiken (Blutgruppe, Rhesusfaktor) Blutflüssigkeit ersetzen können. Reine Salzlösungen (physiologische Kochsalzlösung, Ringerlösung) eignen sich wegen ihrer zu geringen Verweildauer im Gefäßsystem und wegen des fehlenden kolloidosmotischen Drucks nicht zur Schockbehandlung.

Polyvinylpyrrolidon-Lösungen werden schon in großem Maßstab als Blutersatzflüssigkeit verwendet. Diese Polymeren sind wegen ihrer Ähnlichkeit zu Peptiden gut körperverträglich und ähneln bei entsprechenden mittleren Molekulargewichten in ihrem kolloidosmotischen Druck dem des Blutplasma. Wenn ihr Molekulargewicht zu hoch ist, werden sie nicht vollständig ausgeschieden, sondern lagern sich im Reticuloendothel ab. Benutzt man andererseits als Blutersatzflüssigkeit ein Polymeres, das nur Moleküle mit verhältnismäßig niedrigem Molgewicht für eine gute Ausscheidbarkeit durch die Niere enthält, so ist der kolloidosmotische Druck und damit die Wirksamkeit gering. Polyvinylpyrrolidon ist ein sehr inertes Polymeres, da es radikalisch polymerisiert wird und die Polymerkette somit aus C—C-Bindungen besteht. Die C—C-Bindungen werden von den Enzymen des Körpers (Blut, Leber) nicht angegriffen und die Polymeren deshalb nicht abgebaut.

In der DE-OS 27 59 150 und der entsprechenden US-Patentanmeldung Ser. N° 973 798, angemeldet 28.12.1978, sind Vinylpyrrolidonpolymerisate und -copolymerisate beschrieben, die B/BL über Ester, Amid, Harnstoff oder Urethangruppen enthaltende Zwischenglieder zu höheren Molekülen verknüpft sind und sich wegen ihrer biologischen Abbaubarkeit zur Herstellung von Blutersatzflüssigkeiten eignen. In der US-Patentschrift 3 931 111 sind ebenfalls hochmolekulare Produkte als abbaubarer Blutplasmaersatz beschrieben, die jedoch nicht unter Verwendung von Vinylpyrrolidonpolymerisaten hergestellt sind. Da beim enzymatischen Abbau der bekannten Blutersatzmittel Spaltprodukte entstehen können, deren physiologische Unbedenklichkeit bisher nicht erprobt oder nicht erwiesen ist, bestand die Aufgabe, abbaubare, für Blutersatzflüssigkeiten geeignete Polymerisate zu schaffen, deren Spaltprodukte physiologisch akzeptabel sind.

Gelöst wird diese Aufgabe durch Vinylpyrrolidonpolymerisate der allgemeinen Formel

$$[A—B—]_m—A$$

worin bedeuten

A eine Kette von 8 bis ungefähr 1 000 Vinylpyrrolidonmolekülen, die durchschnittlich 2 Hydroxylgruppen enthält,

B den Rest des α,ω-Diisocyanats eines aliphatischen Alkan-1-carbonsäureesters, der 2 bis 11 C-Atome im Alkan- und 2 bis 10 C-Atome im Esterteil hat

m 1 bis 50

und worin die Verknüpfungen zwischen A und B Urethangruppen sind, gebildet einerseits aus den Hydroxylgruppen an der Vinylpyrrolidonkette und andererseits aus den Isocyanatgruppen.

Diese Verbindungen sind wasserlösliche Blockpolymerisate, die überraschenderweise beim biologischen Abbau neben den Vinylpyrrolidonpolymerisaten α,ω-Diaminoalkancarbonsäuren als Spaltprodukte ergeben, obwohl zu erwarten war, daß die Ester nicht abgebaut werden.

Ein Vinylpyrrolidonpolymerisat mit Hydroxylendgruppen kann man erhalten, indem man auf ein durch radikalische Polymerisation von N-Vinylpyrrolidon-2 in Gegenwart von Wasserstoff-peroxid als radikalbildenden Initiator erhaltenes Polymerisat komplexe Hydride einwirken läßt (DE-A-28 31 335, angemeldet am 17.07.1978, offengelegt am 7.02.1980). Diese Vinylpyrrolidonpolymerisate enthalten je Makromolekül 2 Hydroxylendgruppen.

Ausgangsverbindung dafür ist ein Polyvinylpyrrolidon, das nach an sich bekannten Methoden durch Polymerisation von N-Vinylpyrrolidon-2 in Gegenwart von Wasserstoffperoxid in wäßriger Lösung erhalten werden kann. Diese Operationen sind bekannt. Es sei beispielsweise auf die Monographie von W. Reppe « Polyvinylpyrrolidon » Verlag Chemie GmbH, Weinheim/Bergstraße verwiesen. Die Polymerisate werden dann mit den komplexen Hydriden behandelt, wobei — bezogen auf das Gewicht der Polymerisate — 0,1 bis 10, vorzugsweise 0,5 bis 5 % zur Anwendung gelangen. Als Hydride verwendet man vor allem solche, die wie Natrium- und Lithiumboranat wasserlöslich sind, doch läßt sich die Reaktion auch mit anderen wie $NaBH(OCH_3)_3$, $NaAlH_4$, $LiAlH_4$, $NaAlH_2(OCH_2OCH_3)_2$ oder $LiAlH\ [OC(CH_3)_3]$ durchführen. Die stark reaktiven komplexen Hydride werden nur in solchen Mengen eingesetzt, daß die Lactamgruppe des Polyvinylpyrrolidons nicht angegriffen wird. Die Reaktion mit den komplexen Hydriden wird, wenn möglich, in Wasser durchgeführt, was mit Lithium und Natriumboranat realisierbar ist. Bei den anderen verwendet man zweckmäßigerweise Lösungsmittel wie niedere Alkohole, z. B. Methanol, Äthanol, Iso- und n-Propanol, n-Butanol oder tert. Butanol, Äther, wie Dioxan oder Tetrahydrofuran, oder Aromaten, wie Benzol, Toluol oder Xylol. Die Reaktion wird bei Temperaturen von 1 bis 150 °C, vorzugsweise 15 bis 80 °C, durchgeführt. Sie richtet sich nach dem Siedepunkt der Lösungsmittel. Wenn man in wäßrigen oder alkoholischen Lösungsmitteln arbeitet, stellt man im allgemeinen pH-Werte von ca. 7 vor der Reaktion ein. Die Dauer der Reaktion schwankt zwischen 0,5 und 24, vorzugsweise 2 bis 8 Stunden.

Die Hydroxylzahl des für die Herstellung der erfindungsgemäßen Blockpolymerisate benutzten Vinylpyrrolidonpolymerisates soll der Formel

$$\text{gefundene Hydroxylzahl} = 56\,000/M_n \times 2$$

M = Zahlenmittel des Molgewichts
entsprechen.

Ein Vinylpyrrolidonpolymerisat mit Hydroxylgruppen kann man auch durch Copolymerisation von Vinylpyrrolidon mit solchen Mengen Hydroxylgruppen tragendem Monomeren erhalten, daß das Polymerisat durchschnittlich 2 Hydroxylgruppen je Makromolekül enthält. Je nach dem Polymerisationsgrad des Vinylpyrrolidonpolymerisats und dem Molgewicht des Hydroxylgruppen tragenden Monomeren liegt dessen Anteil zwischen 0,1 Gew.% und 12 Gew.%, bezogen auf Vinylpyrrolidon. Beispielsweise sind es bei Allylalkohol und einem Polymerisationsgrad von 9 5,4 Gew.%, bei einem Polymerisationsgrad von 90 0,5 Gew.% ; bei Hydroxypropylmethacrylamid sind die entsprechenden Werte 12,5 bzw. 1,4 Gew.%.

Als Hydroxylgruppen tragende copolymerisierbare Monomere eignen sich z. B. Hydroxyethylacrylat, 2-Hydroxypropylacrylat, Allylalkohol, N-2-Hydroxyethyl-(2meth)acrylamid, N-2-Hydroxypropyl(meth)acrylamid. Der Ausdruck Vinylpyrrolidonpolymerisat umfaßt demnach im vorliegenden Zusammenhang auch derartige Copolymerisate.

Es ist wichtig, daß das Vinylpyrrolidonpolymerisat, das für die Herstellung der erfindungsgemäßen Blockpolymerisate verwendet wird, weitgehend von Verunreinigungen befreit wird, die ihrerseits mit Isocyanaten reagieren würden, wie Wasser, Pyrrolidon, Amine und Säuren. Als Reinigungsmaßnahme empfiehlt sich der Kationen- und Anionenaustausch. Ionenaustauscher sind zum Beispiel unter dem Markenzeichen Lewatit® im Handel.

Das Molgewicht (Zahlenmittel des Molgewichts) des gereinigten Vinylpyrrolidonpolymerisats wird mit dem Dampfdruckosmometer bestimmt. Das durchschnittliche Molgewicht des zur Herstellung der erfindungsgemäßen Blockpolymerisate benutzten Vinylpyrrolidonpolymerisats soll ungefähr von 888 bis ungefähr 111 000 betragen, bevorzugt 1 000 bis 10 000.

Auch für die Bestimmung der Hydroxylzahl nach der oben gezeigten Gleichung ist es wichtig, gereinigtes Vinylpyrrolidonpolymerisat zu verwenden.

$\alpha,\omega$-Diisocyanato-alkancarbonsäureester der bezeichneten Art kann man nach der DE-OS 14 18 995 erhalten. Als

$\alpha,\omega$-Diisocyanatoalkancarbonsäureester können z. B.
2,6-Diisocyanatoethylcapronat,
2,6-Diisocyanatopropylcapronat,
2,6-Diisocyanatobutylcapronat,
2,6-Diisocyanatooctylcapronat,
2,5-Diisocyanatoethylvalerianat und
2,5-Diisocyanatopropylvalerianat verwendet werden. Die entsprechenden Propionsäure-, Buttersäure-, Caprylsäure-, und Dodecancarbonsäure-Derivate seien als weitere Beispiele genannt. Die Alkoholreste im Ester können aus der aliphatischen Reihe vom Ethanol bis zum Decanol ausgewählt werden, wobei Ethanol bis Hexanol bevorzugt sind. Die Capronsäure-Derivate sind bevorzugt, da ihr Abbauprodukt Lysin ist.

Das Mengenverhältnis Vinylpyrrolidonpolymerisat zu Diisocyanat soll unter Berücksichtigung des gewünschten Wertes von m ungefähr stöchiometrisch sein. Zu wenig oder zu viel Diisocyanat ergibt nicht das gewünschte Molekulargewicht des Blockpolymerisats. Im allgemeinen werden auf 100 Gew.-Teile Vinylpyrrolidonpolymerisat

$$\frac{MG_{\text{Isocyanat}} \times 100 \times \text{Hydroxylzahl}}{56\,000 \times 2}$$

Gew.-Teile Diisocyanat (MG = Molgewicht) benötigt, wobei Abweichungen von diesem Wert bis ± 10 % sich im Endergebnis nicht oder fast nicht bemerkbar machen.

Die Umsetzung des Vinylpyrrolidonpolymerisats mit den Diisocyanaten wird zweckmäßigerweise in wasserfreiem Reaktionsmedium bei ungefähr 15 bis 150 °C in Gegenwart üblicher Katalysatoren für die Bildung von Urethanen aus Isocyanaten vorgenommen. Geeignete Lösungsmittel sind beispielsweise aprotische, wie N-Methylpyrrolidon, Dimethylsulfoxid, Dimethylformamid, Toluol, Dioxan, Chlorkohlenwasserstoffe und Gemische dieser Flüssigkeiten. Die bevorzugte Reaktionstemperatur liegt bei ungefähr 25 bis 100 °C.

Durch Dauer und Temperatur der Umsetzung und durch den Reinheitsgrad des Vinylpyrrolidonpolymerisats lassen sich die Werte m zusätzlich regeln, ohne daß der Fachmann hierfür besondere Anweisungen benötigt. Der Wert m läßt sich während der Umsetzung durch übliche Meßmethoden ermitteln.

Als Katalysatoren können z. B. tertiäre Amine oder organische Zinnverbindungen, wie Triethylamin und Dibutylzinndilaurat, benutzt werden.

Die Umsetzung des Vinylpyrrolidonpolymerisats mit den Diisocyanaten kann auch ohne Benutzung von Katalysatoren vorgenommen werden, jedoch muß man dann längere Reaktionszeiten, bei niedrigeren Temperaturen bis zu einigen Tagen, in Kauf nehmen.

Das durch Umsetzung von Vinylpyrrolidonpolymerisat mit Diisocyanat gewonnene Blockpolymerisat kann durch Abdestillieren des Lösungsmittels oder durch Ausfällen in einer nicht lösenden Flüssigkeit, wie Diethylether, gewonnen werden.

Es ist überraschend, daß man auf die beschriebene Weise wasserlösliche Blockpolymerisate erhält, denn man müßte erwarten, daß die Carbonsäureestergruppe im Diisocyanat durch Umesterungsreaktionen zu Vernetzungen führt und daß die hydrophobe Estergruppe im Diisocyanat die Wasserlöslichkeit in unerwünschtem Maße herabsetzt.

Zur Herstellung von Blutersatzflüssigkeiten aus den erfindungsgemäßen Blockpolymerisaten können die üblichen Methoden zur Bereitung pyrogenfreier Flüssigkeiten angewendet werden. Mischungen mit beispielsweise physiologischer Kochsalzlösung oder Ringerlösung sind ebenfalls möglich. Die neuen Blockpolymerisate haben die gleiche Wirksamkeit wie die als Blutersatzmittel üblicherweise verwendeten Polyvinylpyrrolidone.

Darüber hinaus sind sie biologisch abbaubar und ergeben Spaltprodukte, die einerseits durch die Niere ausscheidbar und andererseits ungiftig sind. Wenn man, wie im folgenden Beispiel beschrieben, 2,6-Diisocyanatocapronsäureethylester verwendet, erhält man als Spaltprodukt das lebensnotwendige Lysin.

Beispiel 1

a) 50 Teile (hier wie im folgenden Gewichtsteile) Polyvinylpyrrolidon, hergestellt nach 1 b) mit K-Wert 17 und Hydroxylzahl 48 werden in 300 Teilen einer Mischung von Dioxan/Toluol 75 : 25 gelöst. Vom Lösungsmittel werden 150 Teile abdestilliert. Man läßt auf 80 °C abkühlen. Der pH der Lösung wird mit Triethylamin auf 7,2 eingestellt (mit Wasser verdünnt gemessen). Innerhalb von 5 Stunden werden 5 Teile 2,6-Diisocyanato-capronsäureethylester, gelöst in 20 Teilen Dioxan, zugeführt. Man läßt noch 10 Stunden bei 80 °C rühren und fällt dann das Polymere durch Eingießen der Reaktionslösung in 1 000 Teile Diethylether aus. Das Blockpolymere hat einen K-Wert von 35,6 (gemessen 5 %ig in Wasser), was einem rechnerischen Molgewicht von 85 700 entspricht.

b) 750 Teile Vinylpyrrolidon werden in 250 Teilen Wasser gelöst, mit 0,5 Teilen 0,01 %iger Kupfer-IIchloridlösung und mit 30 Teilen 30 %igem Wasserstoffperoxid versetzt und 6 Stunden bei 70 °C bei einem pH-Wert von 7,6 polymerisiert. Die Polymerisatlösung wird gefriergetrocknet. Das erhaltene Polymerisat weist einen K-Wert von 17 und eine Hydroxylzahl 38 auf und hat einen Pyrrolidongehalt von 5,9 %.

100 Teile dieses Polymerisats werden in 160 Teilen Wasser gelöst, mit 10 Raumteilen Ammoniak auf pH 7,5 eingestellt und portionsweise mit 2 Teilen Natriumboranat versetzt. Während des Schäumens (ca. 1 Stunde) wird bei Raumtemperatur gerührt. Man läßt über Nacht stehen und reinigt über 2 000 Teilen Lewatit S 100 und 2 000 Teilen Lewatit M 500. Anschließend wird gefriergetrocknet. Das Polymerisat hat die Hydroxylzahl 48 und enthält 1,3 % Pyrrolidon.

Beispiel 2

a) Das Blockpolymere aus 1a) wird als 1,5 %ige Lösung in einem Gemisch Blutplasma/Wasser 1 : 1 drei Tage lang bei pH 7,2 und 37 °C gerührt und die Mischung anschließend gefriergetrocknet. Man schlämmt in einem Gemisch aus Methanol/Methylenchlorid 1 : 3 auf, läßt 20 min stehen und filtriert ab. Das Filtrat wird eingeengt und das Festprodukt in $CH_2Cl_2$ aufgenommen. Es wird mit Diethylether ausgefällt, abfiltriert und getrocknet. Das Produkt hat einen K-Wert von 27,4 (5 %ig in Wasser), was einem rechnerischen Molgewicht von 39 000 entspricht.

b) Erhitzt man das Blockpolymere aus 1a) in wäßriger Lösung mit 5 % NaOH 3 Tage lang bei 100 °C und bestimmt durch Dünnschichtchromatographie die Hydrolyseprodukte, so kann man Lysin als Abbauprodukt nachweisen.

Dünnschichtchromatographie der Abbauprodukte aus 2a) gibt keine sichere Aussage, da das Blutplasma selbst Lysin enthält.

c) Rührt man das Polymerisat als 1,5 %ige wäßrige Lösung 3 Tage bei pH 7,2 und 37 °C und gefriertrocknet, so hat das Produkt einen K-Wert von 35,2 (5 %ig in Wasser). Damit wird bei Vergleich mit dem K-Wert von Beispiel 1a deutlich, daß das Blockpolymerisat durch Behandeln in Wasser nicht abgebaut wird.

**Ansprüche**

1. Vinylpyrrolidonpolymerisate der allgemeinen Formel

$$[A—B—]_m—A$$

worin bedeuten

A eine Kette von 8 bis 1 000 Vinylpyrrolidonmolekülen, die durchschnittlich 2 Hydroxylgruppen enthält,

B den Rest des α,ω-Diisocyanats eines aliphatischen Alkan-1-carbonsäureesters, der 2 bis 11 C-Atome im Alkan- und 2 bis 10 C-Atome im Esterteil hat

m 1 bis 50

und worin die Verknüpfungen zwischen A und B Urethangruppen sind, gebildet einerseits aus den Hydroxylgruppen an der Vinylpyrrolidonkette und andererseits aus den Isocyanatgruppen.

2. Vinylpyrrolidonpolymerisate gemäß Anspruch 1, deren Vinylpyrrolidonteil dadurch gewonnen wurde, daß man auf ein durch radikalische Polymerisation von N-Vinyl-pyrrolidon-2 in Gegenwart von Wasserstoffperoxid als radikalbildenden Initiator erhaltenes Polymerisat komplexe Hydride einwirken läßt.

3. Vinylpyrrolidonpolymerisate des Anspruchs 1, dadurch gekennzeichnet, daß der Vinylpyrrolidonteil A durch Copolymerisation von Vinylpyrrolidon mit solchen (0,1 bis 12 Gewichtsprozent) Mengen Hydroxylgruppen tragendem Monomeren erhalten worden ist, daß das Polymerisat durchschnittlich 2 Hydroxylgruppen je Makromolekül enthält.

4. Verfahren zur Herstellung von Vinylpyrrolidonpolymerisaten gemäß Anspruch 1, dadurch gekennzeichnet, daß man ein Vinylpyrrolidonpolymerisat aus 8 bis 1 000 Vinylpyrrolidonmolekülen, das durchschnittlich 2 Hydroxylgruppen je Makromolekül enthält, mit den zum Wert m führenden Mengen des α,ω-Diisocyanats eines aliphatischen Alkan-1-carbonsäureesters, der 2 bis 11 C-Atome im Alkan- und 2 bis 10 C-Atome im Esterteil hat, umsetzt.

5. Verwendung der Polymerisate gemäß Anspruch 1 bis 3 in Blutersatzflüssigkeiten.

6. Blutersatzflüssigkeiten, enthaltend Vinylpyrrolidonpolymerisate gemäß Anspruch 1.

**Claims**

1. A vinylpyrrolidone polymer of the general formula

$$[—A—B—]_m—A$$

where

A is a chain of from 8 to 1,000 vinylpyrrolidone molecules containing on average 2 hydroxyl groups,

B is the radical of the α,ω-diisocyanate of an aliphatic alkane-1-carboxylic acid ester of 2 to 11 carbon atoms in the alkane moiety and of 2 to 10 carbon atoms in the ester moiety and

m is from 1 to 50,

the linkage between A and B being urethane groups formed from the hydroxyl groups on the vinylpyrrolidone chain and from the isocyanate groups.

2. A vinylpyrrolidone polymer as claimed in claim 1, wherein the vinylpyrrolidone component has been produced by treating a polymer, obtained by free radical polymerization of N-vinylpyrrolid-2-one in the presence of hydrogen peroxide as a free radical initiator, with a complex hydride.

3. A vinylpyrrolidone polymer as claimed in claim 1, wherein the vinylpyrrolidone component A has been obtained by copolymerizing the vinylpyrrolidone with such an amount (0.1 to 12 % by weight) of a hydroxyl-containing monomer that the polymer contains an average of 2 hydroxyl groups per macromolecule.

4. A process for the preparation of a vinylpyrrolidone polymer as claimed in claim 1, wherein a vinylpyrrolidone polymer containing from 8 to 1,000 vinylpyrrolidone molecules and an average of 2 hydroxyl groups per macromolecule is reacted with an α,ω-diisocyanate of an aliphatic alkane-1-carboxylic acid ester of 2 to 11 carbon atoms in the alkane moiety and 2 to 10 carbon atoms in the ester moiety, in an amount which gives the value m.

5. Use of a polymer as claimed in claims 1 to 3 in a plasma substitute.

6. A plasma substitute which contains a vinylpyrrolidone polymer as claimed in claim 1.

**Revendications**

1. Polymères de vinylpyrrolidone de formule générale

$$(A—B—)_m—A$$

dans laquelle

A représente une chaîne de 8 à 1 000 molécules de vinylpyrrolidone contenant en moyenne 2 groupes hydroxyle

B est le reste du α,ω-diisocyanate d'un ester d'acide alcane-1-carboxylique aliphatique qui possède 2 à 11 atomes dans la partie alcane et 2 à 10 atomes C dans la partie ester

m = 1 à 50

et où les liaisons entre A et B sont des groupes uréthane, formés d'une part, des groupes hydroxyle sur la chaîne vinylpyrrolidone et, d'autre part, des groupes isocyanate.

2. Polymères de vinylpyrrolidone selon la revendication 1, dont la partie vinylpyrrolidone a été obtenue en faisant agir un hydrure complexe sur un polymère obtenu par polymérisation radicalaire de N-vinylpyrrolidone-2, en présence de peroxyde d'hydrogène comme initiateur de formation de radicaux.

3. Polymères de vinylpyrrolidone de la revendication 1, caractérisés par le fait que la partie vinylpyrrolidone A a été obtenue par copolymérisation de vinylpyrrolidone avec une quantité (0,1 à 12 % en poids) de monomères, porteurs de groupes hydroxyle, telle que le polymère contienne en moyenne 2 groupes hydroxyle par macromolécule.

4. Procédé de préparation de polymères de vinylpyrrolidone selon la revendication 1, caractérisé par le fait qu'on fait réagir un polymère de vinylpyrrolidone de 8 à 1 000 molécules de vinylpyrrolidone, qui contient en moyenne 2 groupes hydroxyle par macromolécule, avec la quantité, conduisant à la valeur m, de α,ω-diisocyanate d'un ester d'acide alcane-1-carboxylique aliphatique qui possède 2 à 11 atomes C dans la partie alcane et 2 à 10 atomes C dans la partie ester.

5. Utilisation des polymères selon la revendication 1 à 3 dans des succédanés de sérum sanguin.

6. Succédanés de sérum sanguin contenant des polymères de vinylpyrrolidone selon la revendication 1.